# EUROPÄISCHE PATENTSCHRIFT

(11) **EP 0 893 430 B1**
(45) Veröffentlichungstag und Bekanntmachung des Hinweises auf die Patenterteilung: **02.10.2002**
(21) Anmeldenummer: 98113686.4
(22) Anmeldetag: 22.07.1998
(51) Int. Cl.: C07C 209/26, C07D 295/023, C07D 295/02

(54) **Verfahren zur Herstellung von Alkylaminen**
Process for the preparation of alkylamines
Procédé pour la préparation d'alkylamines

(30) Priorität: 23.07.1997 DE 19731745
(43) Veröffentlichungstag der Anmeldung: 27.01.1999
(73) Patentinhaber: BASF AKTIENGESELLSCHAFT, 67056 Ludwigshafen (DE)
(72) Erfinder: Kneuper, Hein-Josef, Dr., 68163 Mannheim (DE); Lebkücher, Rolf Dr., 68165 Mannheim (DE); Neuhauser, Horst, Dr., 67373 Dudenhofen (DE); Henne, Andreas, Dr., 67433 Neustadt (DE); Becker, Rainer, Dr., 67098 Bad Dürkheim (DE)
(74) Vertreter: Isenbruck, Günter, Dr.

(56) Entgegenhaltungen:
- EP-A- 0 297 295
- EP-A- 0 435 072
- EP-A- 0 492 771
- FR-A- 2 349 565
- US-A- 3 249 613

## Beschreibung

Die Erfindung betrifft ein Verfahren zur Herstellung von Alkylaminen.

Die Herstellung von Alkylaminen durch Umsetzung von Aminen mit Aldehyden zu Alkanolaminen, die anschließend zu Alkylaminen hydriert werden, ist bekannt. In der US 3,249,613 ist beispielsweise die Umsetzung von Piperazin mit Formaldehyd zu 1,4-Piperazindimethanol beschrieben, wobei anschließend das 1,4-Piperazindimethanol zu 1,4-Dimethylpiperazin unter Verwendung eines metallischen Hydrierungskatalysators hydriert wird. Entsprechende Umsetzungen sind in der DE-A-25 31 060 beschrieben.

Vorteilhaft ist eine kontinuierliche Verfahrensführung bei der Herstellung der Alkylamine. Ein Vermischen von eingesetzten Aminen und Aldehyden führt jedoch zur Bildung von polymeren Kondensationsprodukten, die in allen gängigen Lösungsmitteln unlöslich sind, siehe W. T. Forsee, Jr. C. B. Pollard, J. Am. Chem. Soc. 57, 1935, 2363 bis 2364. Eine Umsetzung und Hydrierung des polymeren Kondensationsproduktes ist nicht möglich. Daher wurde versucht, durch spezielle Verfahrensweisen dieses Problem zu umgehen.

Auch ein Verfahren zur kontinuierlichen Herstellung von Methylaminen aus Aminen, Formaldehyd und Wasserstoff ist bekannt. In der EP-A-0 297 295 ist ein entsprechendes Verfahren beschrieben, bei dem Amine, Formaldehyd und Wasserstoff unter Druck und erhöhter Temperatur in einem Festbettkatalysator in flüssiger Phase in einem Schritt zu Methylaminen umgesetzt werden. Es werden insbesondere N,N-Dimethyl-n-octylamin, N,N-Dimethylanilin, N,N-Dimethylpiperazin und N,N-Dimethyldiglykolamin auf diese Wese hergestellt.

Um die Zersetzung des Katalysators zu verhindern, wird gemäß EP-A-0 297 295 der Wassergehalt des im Reaktor vorliegenden Gemisches begrenzt. Die Summe der Wasseranteile der zugeführten Ströme soll einen Wert von 50 Gew. - % nicht übersteigen. Insbesondere soll der Wassergehalt der Formaldehydlösung 7 bis 15 Gew.-% betragen. Zudem wird es als entscheidend herausgestellt, daß Aminstrom und Formaldehydstrom voneinander getrennt auf eine vorgegebene Temperatur aufgeheizt werden, indem sie über eigene Leitungen geführt und erwärmt werden, bevor die Reaktion durchgeführt wird.

Nachteil des Verfahrens ist der hohe apparative Aufwand für die getrennte Vorheizung der Reaktionsteilnehmer und die Notwendigkeit des Verwendens einer konzentrierten Formaldehydlösung. Üblicherweise wird Formaldehyd als etwa 30 Gew. - %ige Lösung in Wasser gehandelt. Eine Aufkonzentrierung des Formaldehydgehalts vor der Umsetzung führt zu erhöhtem Aufwand und hohen Kosten des Verfahrens.

FR-A-2 349 565 (DE-A-26 18 580) betrifft die Herstellung von tertiären Aminen durch Umsetzung von Aminen mit Formaldehyd, wobei die Reaktion in Gegenwart von freiem Wasserstoff an einem Festbettkatalysator durchgeführt wird. Amine und Formaldehyd werden getrennt in den Reaktor eingerührt, um die Bildung von polymeren Kondensationsprodukten während der Reaktion zu vermeiden.

Aufgabe der vorliegenden Erfindung ist die Bereitstellung eines Verfahrens zur Herstellung von Alkylaminen, das die vorstehenden Nachteile vermeidet und insbesondere bei vereinfachtem technischem Aufbau wirtschaftlich durchrührbar ist. Als Ausgangsstoffe sollen handelsübliche Produkte einsetzbar sein.

Die Aufgabe wird erfindungsgemäß gelöst durch ein Verfahren zur Herstellung von Alkylaminen durch Umsetzung von Aminen und aliphatischen Aldehyden in Gegenwart von freiem Wasserstoff an einem Festbettkatalysator in einem Reaktor, in dem Amine und Aldehyde nach Zuführung in getrennten Strömen im Katalysatorbett gemischt werden, wobei nur der Aldehydstrom oder nur der Aminstrom bei der Zuführung vorgeheizt wird.

Es wurde erfindungsgemäß gefunden, daß Alkylamine durch Umsetzung von Aminen und aliphatischen Aldehyden in Gegenwart von freiem Wasserstoff kontinuierlich hergestellt werden können, wenn lediglich einer der Ströme für die Reaktanden bei der Zuführung vorgeheizt wird. Zudem ist das Verfahren auch unter Verwendung von wasserhaltigen Reaktanden durchführbar, wobei der Wassergehalt hoch sein kann. Dies führt nicht zu einer Schädigung des eingesetzten Festbettkatalysators.

Im erfindungsgemäßen Verfahren kann beispielsweise handelsüblicher Formaldehyd eingesetzt werden, der in der Regel als 30 bis 40 Gew.-%ige Lösung in Wasser vertrieben wird. Eine bei bekannten Verfahren benötigte Aufkonzentrierung des Formaldehyds kann entfallen. Zudem kann die zur Herstellung der Alkylamine verwendete Apparatur vereinfacht werden, indem nur ein Reaktandenstrom vorgeheizt werden muß. Da das Verfahren in der Regel unter Hochdruckbedingungen durchgeführt wird, müssen die zum Vorheizen eingesetzten Wärmetauscher den Hochdruckbedingungen genügen. Derartige Wärmetauscher sind kostspielig.

Erfindungsgemäß eingesetzte Amine weisen vorzugsweise mindestens eine sekundäre Aminogruppe auf. Besonders bevorzugt handelt es sich um sekundäre Amine mit 1 bis 3 Aminogruppen. Beispiele geeigneter Amine sind Diethylentriamin, Dipropylentriamin, Morpholin, Piperidin, Pyrrolidin, Piperazin, 1-Methylpiperazin. Insbesondere wird Piperazin im erfindungsgemäßen Verfahren eingesetzt. Die eingesetzten Amine können ohne Zusatz von Lösungsmitteln oder in Form von Lösungen eingesetzt werden. Vorzugsweise werden keine Alkohole als Lösungsmittel eingesetzt. Gegebenenfalls können Alkohole in geringen Mengen aus den eingesetzten Aldehyden gebildet werden. Es liegen vorzugsweise insgesamt keine Alkohole im Reaktionssystem vor. Besonders bevorzugt werden die Amine als wäßrige Lösungen eingesetzt. Beispielsweise wird Piperazin als 30 bis 80 Gew.-%ige Lösung in Wasser eingesetzt. Insbesondere beträgt die Piperazin-Konzentration 60 bis 70 Gew.-%.

Die erfindungsgemäß eingesetzten aliphatischen Aldehyde können eine oder mehrere Aldehydfunktionen aufweisen. Vorzugsweise handelt es sich um Mono- oder Dialdehyde, insbesondere um Monoaldehyde. Die Aldehyde weisen dabei vorzugsweise 1 bis 10, besonders bevorzugt 1 bis 3, insbesondere 1 oder 2 Kohlenstoffatome auf. Speziell bevorzugt wird Formaldehyd eingesetzt.

Die Aldehyde werden ebenfalls vorzugsweise als wäßrige Lösungen eingesetzt. Dabei beträgt der Wasseranteil in der Aldehydlösung zwischen 50 und 90, vorzugsweise zwischen 50 und 80, insbesondere zwischen 60 und 80, speziell zwischen 65 und 75 Gew.-%, bezogen auf das Gesamtgewicht der Lösung.

Die Umsetzung der Amine mit den aliphatischen Aldehyden führt zu einer Alkylsubstitution am Stickstoffatom, wobei der eingeführte Alkylrest eine Anzahl an Kohlenstoffatomen aufweist, die der Anzahl an Kohlenstoffatomen im Aldehyd entspricht. Bei der Verwendung von Formaldehyd wird somit eine Methylierung durchgeführt. Vorzugsweise werden die bevorzugt eingesetzten sekundären Amine peralkyliert beziehungsweise permethyliert. Somit wird das speziell bevorzugte Piperazin zu 1,4-Dimethylpiperazin umgesetzt.

Dabei sollte der Anteil an monoalkylierten Verbindungen, speziell N-Methylpiperazin, möglichst gering gehalten werden.

Die dem Reaktor zugeführten Reaktandenströme können einen hohen Wasseranteil aufweisen, ohne daß es zu einer Schädigung des Reaktors oder Katalysators kommt. Beispielsweise kann die Summe der Wasseranteile der dem Reaktor zugeführten Ströme der Amin- und Aldehydlösungen zwischen 50 und 80, vorzugsweise zwischen 50 und 70, insbesondere zwischen 55 und 70, speziell zwischen 60 und 70 Gew.-%, bezogen auf das Gesamtgewicht der Ströme, betragen.

Das Molverhältnis der bevorzugten Reaktanden Piperazin und Formaldehyd beträgt vorzugsweise 1 : 1,0 bis 1 : 5, besonders bevorzugt 1 : 1,5 bis 1 : 3, insbesondere 1 : 2 bis 1 : 2,7.

Die Umsetzung wird an einem Festbettkatalysator durchgeführt. Dabei können alle geeigneten Festbettkatalysatoren eingesetzt werden. Vorzugsweise ist der Festbettkatalysator ein trägerfreier Katalysator, der in oxidischer Form mindestens 60 Gew.-% CoO und 10 bis 30 Gew.-% CuO, bezogen auf das Gesamtgewicht des Katalysators, enthält, wobei bis zu einem Drittel der Menge des CoO durch NiO ersetzt sein kann.

Der Katalysator kann dabei in geringeren Mengen noch weitere Stoffe enthalten, beispielsweise Chrom, Aluminium, Silber, Alkali- und/oder Erdalkalimetalle oder deren Verbindungen, Vanadium, Wolfram, Bor oder deren Verbindungen, Edelmetalle der Platinreihe, Phosphorsäure und besonders bevorzugt Mangan und Molybdän oder deren Verbindungen. Außerdem kann der Katalysator geringe Mengen an gewöhnlich als Trägerstoffe eingesetzten Substanzen enthalten, ohne daß der Charakter des Vollkontaktes verloren geht. Ein besonders bevorzugter Katalysator enthält in der oxidischen Form CoO, CuO, Mn₃O₄ und MoO₃. Insbesondere besteht er im wesentlichen (mehr als 90 Gew.-%) oder vollständig aus diesen Inhaltsstoffen. Ein insbesondere bevorzugter Katalysator weist etwa die folgende Zusammensetzung auf: 60 % bis 70 % CoO, 15 % bis 25 % CuO, 5 % bis 10 % Mn₃O₄ und 0 % bis 0 % MoO₃. Die Prozentangaben sind dabei jeweils Gew.-%.

Derartige Katalysatoren sind beispielsweise in DE-A-26 18 580 beschrieben. Auch Verfahren zur Herstellung der Katalysatoren sind in dieser Druckschrift beschrieben.

Das erfindungsgemäße Verfahren wird vorzugsweise bei einer Temperatur von 60 bis 200°C, besonders bevorzugt 80 bis 160°C durchgeführt. Dabei beträgt der Gesamtdruck vorzugsweise 10 bis 700, besonders bevorzugt 100 bis 300 bar.

Der verwendete Reaktor ist dabei vorzugsweise ein Rohrreaktor, der beispielsweise in Sumpf- oder Rieselfahrweise betrieben werden kann. Im erfindungsgemäßen Verfahren wird dabei vorzugsweise die wäßrige Piperazinlösung durch einen Wärmetauscher auf eine Temperatur von 100°C erhitzt und bei dieser Temperatur in den Rohrreaktor geführt. Die wäßrige Formaldehydlösung wird ohne vorheriges Aufheizen ebenfalls in den Rohrreaktor geführt, wobei die Zuführung im Katalysatorbett endet. Hierdurch wird sichergestellt, daß eine Vermischung der Reaktanden erst im Katalysatorbett erfolgt. Es ist auch möglich, den Reaktor anders aufzubauen, solange sicher gestellt ist, daß keine nennenswerte Durchmischung der Amine und Aldehyde vor dem Kontakt mit dem Katalysator erfolgt.

Durch das erfindungsgemäße Verfahren können Umsätze von mehr als 99,9 % und Selektivitäten von mehr als 99,8 % erreicht werden, wobei der Rückstand weniger als 0,5 Gew.-% ausmacht.

Die Erfindung wird nachstehend anhand von Beispielen näher erläutert.

### Vergleichsbeispiel V1

Eine 60 Gew.-%ige Lösung von Piperazin in Wasser und eine 30 Gew.-%ige Lösung von Formaldehyd in Wasser wurden bei Raumtemperatur vermischt. Es bildete sich ein weißer Feststoff, der in keinem gängigen Lösungsmittel löslich war.

### Beispiel 1

Ein Rohrreaktor wurde mit 2 getrennten Zulaufleitungen so ausgerüstet, daß er in Rieselfahrweise betrieben werden konnte. Die Zulaufleitung für Formaldehyd wurde dabei bis auf das Katalysatorbett geführt. Der Piperazin/-Wasser-Zulauf wurde vor dem Erreichen des Katalysatorbettes auf eine Temperatur von etwa 100°C vorgeheizt. Der eingesetzte Katalysator wies etwa folgende Zusammensetzung auf (in Gew.-%): 65 % CoO, 20 % CuO, 10 % Mn₃O₄, 5 % MoO₃. Die Umsetzung wurde bei einer Temperatur von 100°C und einem Wasserstoffdruck von 200 bar durchgeführt. Dabei betrug die Katalysatorbelastung 0,12 l/l h, bezogen auf Piperazin. Das Molverhältnis von Piperazin zu Formaldehyd betrug 1 : 2,3. Der Rohrreaktor konnte für mehr als 12 Tage ohne Auftreten einer Verstopfung betrieben werden. Umsatz und Selektivität betrugen jeweils mehr als 99,8 %, wobei weniger als 0,5 Gew.-% Rückstand erhalten wurden. Eine Schädigung oder Desaktivierung des Katalysators konnte nicht festgestellt werden.

### Beispiel 2

Beispiel 1 wurde wiederholt, jedoch wurde die Formaldehydzuführung in Höhe des Reaktorkopfes abgetrennt und beide Zuläufe an gegenüberliegenden Seiten der Reaktorwand auf das Katalysatorbett geführt. Der Reaktor konnte für mehr als 4 Tage mit dem gleichen Ergebnis wie in Beispiel 1 angegeben betrieben werden.

## Patentansprüche

1. Verfahren zur Herstellung von Alkylaminen durch Umsetzung von Aminen und aliphatischen Aldehyden in Gegenwart von freiem Wasserstoff an einem Festbettkatalysator in einem Reaktor, in dem Amine und Aldehyde nach Zuführung in getrennten Strömen im Katalysatorbett gemischt werden, **dadurch gekennzeichnet, daß** nur der Aldehydstrom oder nur der Aminstrom bei der Zuführung vorgeheizt wird.

2. Verfahren nach Anspruch 1, **dadurch gekennzeichnet, daß** der Aminstrom, nicht jedoch der Aldehydstrom vorgeheizt wird.

3. Verfahren nach Anspruch 1 oder 2, **dadurch gekennzeichnet, daß** Amine und/oder Aldehyde als wäßrige Lösungen eingesetzt werden.

4. Verfahren nach Anspruch 3, **dadurch gekennzeichnet, daß** die wäßrige Lösung des Aldehyds zwischen 50 und 90 Gew.-% Wasser, bezogen auf das Gesamtgewicht der Lösung, enthält.

5. Verfahren nach Anspruch 3 oder 4 **dadurch gekennzeichnet, daß** die Summe der Wasseranteile der dem Reaktor zugeführten Ströme der Amin- und Aldehydlösungen zwischen 50 und 80 Gew.-%, bezogen auf das Gesamtgewicht der Ströme, beträgt.

6. Verfahren nach einem der Ansprüche 1 bis 5, **dadurch gekennzeichnet, daß** der Festbettkatalysator ein trägerfreier Katalysator ist, der in oxidischer Form mindestens 60 Gew.-% CoO und 10 bis 30 Gew.-% CuO, bezogen auf das Gesamtgewicht des Katalysators, enthält, wobei bis zu ein Drittel der Menge des CoO durch NiO ersetzt sein kann.

7. Verfahren nach einem der Ansprüche 1 bis 6, **dadurch gekennzeichnet; daß** das Amin ein sekundäres Amin mit 1 bis 3 Aminogruppen ist.

8. Verfahren nach Anspruch 7, **dadurch gekennzeichnet, daß** das Amin Piperazin ist.

9. Verfahren nach einem der Ansprüche 1 bis 8, **dadurch gekennzeichnet, daß** der Aldehyd Formaldehyd ist.

10. Verfahren nach einem der Ansprüche 1 bis 9, **dadurch gekennzeichnet, daß** die Umsetzung zu peralkylierten Aminen führt.

## Claims

1. A process for preparing alkylamines by reacting amines and aliphatic aldehydes in the presence of free hydrogen on a fixed bed catalyst in a reactor in which amines and aldehydes are mixed in the catalyst bed after being fed in in separate streams, wherein only the aldehyde stream or only the amine stream is preheated during the feeding in.

2. A process as claimed in claim 1, wherein the amine stream, but not the aldehyde stream, is preheated.

3. A process as claimed in claim 1 or 2, wherein amines and/or aldehydes are employed as aqueous solutions.

4. A process as claimed in claim 3, wherein the aqueous solution of the aldehyde contains from 50 to 90% by weight of water based on the total weight of the solution.

5. A process as claimed in claim 3 or 4, wherein the total of the water contents of the streams of the amine and aldehyde solutions fed into the reactor is from 50 to 80% of the total weight of the streams.

6. A process as claimed in any of claims 1 to 5, wherein the fixed bed catalyst is an unsupported catalyst which, in the oxide form, contains at least 60% by weight of CoO and from 10 to 30% by weight of CuO, based on the total weight of the catalyst, it being possible for up to one third of the amount of CoO to be replaced by NiO.

7. A process as claimed in any of claims 1 to 6, wherein the amine is a secondary amine having 1 to 3 amino groups.

8. A process as claimed in claim 7, wherein the amine is piperazine.

9. A process as claimed in any of claims 1 to 8, wherein the aldehyde is formaldehyde.

10. A process as claimed in any of claims 1 to 9, wherein the reaction results in peralkylated amines.

## Revendications

1. Procédé de préparation d'alkylamines par réaction d'amines et d'aldéhydes aliphatiques en présence d'hydrogène libre sur un catalyseur à lit fixe, dans un réacteur, dans lequel amines et aldéhydes sont mélangés après amenée en courants séparés dans le lit de catalyseur, **caractérisé en ce que** seul le courant d'aldéhyde ou seul le courant d'amine est préchauffé lors de l'amenée.

2. Procédé suivant la revendication 1, **caractérisé en ce que** le courant d'amine, mais non pas le courant d'aldéhyde, est préchauffé.

3. Procédé suivant l'une des revendications 1 et 2, **caractérisé en ce que** les amines et/ou les aldéhydes sont mis en oeuvre sous la forme de solutions aqueuses.

4. Procédé suivant la revendication 3, **caractérisé en ce que** la solution aqueuse de l'aldéhyde contient entre 50 et 90% en poids d'eau, par rapport au poids total de la solution.

5. Procédé suivant l'une des revendications 3 et 4, **caractérisé en ce que** la somme des fractions d'eau des courants, amenés au réacteur, des solutions d'amine et d'aldéhyde est comprise entre 50 et 80% en poids, par rapport au poids total des courants.

6. Procédé suivant l'une des revendications 1 à 5, **caractérisé en ce que** le catalyseur à lit fixe est un catalyseur exempt de support, qui contient, sous forme oxydée, au moins 60% en poids de CoO et 10 à 30% en poids de CuO, par rapport au poids total du catalyseur, jusqu'à un tiers de la quantité du CoO pouvant être remplacé par NiO.

7. Procédé suivant l'une des revendications 1 à 6, **caractérisé en ce que** l'amine est une amine secondaire comportant 1 à 3 groupes amino.

8. Procédé suivant la revendication 7, **caractérisé en ce que** l'amine est de la pipérazine.

9. Procédé suivant l'une des revendications 1 à 8, **caractérisé en ce que** l'aldéhyde est du formaldéhyde.

10. Procédé suivant l'une des revendications 1 à 9, **caractérisé en ce que** la réaction conduit à des amines peralkylées.
